# EUROPÄISCHE PATENTSCHRIFT

(11) **EP 2 774 909 B1**
(45) Veröffentlichungstag und Bekanntmachung des Hinweises auf die Patenterteilung: **12.08.2015**
(21) Anmeldenummer: 14155738.9
(22) Anmeldetag: 19.02.2014
(51) Int. Cl.: C07C 37/055

(54) **Verfahren zur Herstellung von Hydroxytyrosol**
Process for the preparation of hydroxytyrosol
Procédé de fabrication d'hydroxytyrosol

(30) Priorität: 05.03.2013 DE 102013203753
(43) Veröffentlichungstag der Anmeldung: 10.09.2014
(73) Patentinhaber: Wacker Chemie AG, 81737 München (DE)
(72) Erfinder: Krüger, Benno, 84508 Burgkirchen (DE); Fleischmann, Gerald, 84489 Burghausen (DE); Petersen, Hermann, 84489 Burghausen (DE)
(74) Vertreter: Potten, Holger

(56) Entgegenhaltungen:
- WO-A1-2012/006783

## Beschreibung

Die Erfindung betrifft ein Verfahren zur Herstellung von Hydroxytyrosol (3,4-Dihydroxyphenylethanol).

Hydroxytyrosol ist ein wirksames Antioxidans und hat aufgrund seiner für die Gesundheit positiven Effekte in den letzten Jahren großes Interesse geweckt. Hydroxytyrosol gilt als aktive Komponente der Mediterranen Diät. Von der European Food Safety Authority (EFSA) wurden Polyphenole aus Oliven mit einem positiven Health Claim belegt, wobei eine tägliche Hydroxytyrosol-Dosis von mindestens 5 mg empfohlen wird. Auch ein entzündungshemmender Effekt von Hydroxytyrosol ist beschrieben worden. Außerdem existieren Studien, die zeigen, dass Hydroxytyrosol in vitro antimikrobielle Eigenschaften gegen Erreger der Atemwege und des Gastrointestinaltrakts aufweist, wie gegen einige Stämme der Gattung Vibrio, Salmonella oder Staphylococcus und dass die eingesetzten Dosen durchaus mit denen von Antibiotika, z.B. Ampicillin, konkurrieren können. Der Substanz wird zudem ein neuroprotektiver und ein anti-proliferativer und proapoptotischer Effekt zugewiesen. Diese Eigenschaften machen Hydroxytyrosol zu einem sehr interessanten und gesuchten Stoff, der in Pharmazeutika, Nahrungsergänzungsmitteln, funktionellen Lebensmitteln oder auch in Kosmetik Anwendungen findet.

Hydroxytyrosol, welches bisher auf dem Markt erhältlich ist, stammt zum großen Teil aus Oliven, Olivenblättern oder Abwasser, das bei der Olivenölherstellung anfällt und wird in Form eines Extraktes angeboten: Der Anteil an Hydroxytyrosol in diesen Produkten ist meist sehr gering. Beispiele hierfür sind HIDROX™ mit einem Hydroxytyrosol-Gehalt, der unter 12% liegt, oder OPEXTAN™, welches ca. 4,5% Hydroxytyrosol enthält.

Neben dem Isolieren von natürlichem Hydroxytyrosol aus Oliven sind zahlreiche Verfahren beschrieben, diesen Stoff synthetisch herzustellen. So beschreibt WO 2008/107109 ein Verfahren zur Herstellung durch Reduktion von 4-(Chloracetyl)-1,2-dihydroxy-benzol (4-(Chloracetyl)catechol) mit Hilfe von Katalysatoren wie Palladium/Kohle. Die Ausgangsverbindung 4-(Chloracetyl)-catechol benötigt zur Herstellung jedoch hohe Temperaturen und lange Reaktionszeiten.

WO 2007/009590 A1 beschreibt ein Verfahren zur Herstellung von Hydroxytyrosol über 3,4-Dihydroxymandelsäure, welche durch Metallkatalysatoren wie Palladium/Kohle zu 3,4-Dihydroxyphenylessigsäure hydriert wird. Anschließend erfolgt die Reduktion zu Hydroxytyrosol. Das gewonnene Hydroxytyrosol besitzt gemäß den Beispielen Reinheiten zwischen 67,9% und 93,8%. Die aus 3,4-Dihydroxymandelsäureester gewonnene Vorstufe 3,4-Dihydroxy-phenylessigsäuremethylester ist bis auf ein Beispiel, in dem ohne Angabe der Reinausbeute durch Umkristallisation ein Produkt mit einer Reinheit von 98% beschrieben ist, nur als Produkt mit Reinheiten zwischen 51,2% und 83,5% beschrieben.

Der Abstract zu KR 2007 038702 A beschreibt eine Synthese über Styroloxidderivate. Die Ausgangssubstanz wird in Gegenwart eines Edelmetallkatalysators wie Palladium auf Aktivkohle hydriert. Epoxide sind hinsichtlich mutagener oder cancerogener Wirkung bedenklich, so dass Spuren im Endprodukt für die Verwendung im Lebensmittelbereich problematisch sind.

Bei der genannten Hydrierungsreaktion werden Ester oder Säureanaloge des Hydroxytyrosols reduziert. Nachteiligerweise werden dazu Edelmetallkatalysatoren oder toxische Katalysatoren wie Nickel benötigt.

WO 2008/110908 A1 beschreibt ein Verfahren ausgehend von Tyrosol. In dem Verfahren wird zunächst die Hydroxyethylgruppe mittels unterschiedlicher Reagenzien geschützt und anschließend mit Derivaten der Iodbenzoesäure in die hydroxyethylgeschützten Tyrosolderivate eine zweite Hydroxygruppe in den aromatischen Ring eingeführt. Sowohl das Ausgangsmaterial Tyrosol als auch die Oxidationsmittel sind sehr hochpreisige Verbindungen. Die Reaktion ist aufgrund der vielen Einsatzstoffe komplex. Es gibt keine Angaben zur Reinheit der nach den unterschiedlichen Verfahren gewonnenen Hydroxytyrosole.

WO 2009/153374 beschreibt ein Herstellverfahren ausgehend von Safrol. Sowohl Safrol als auch das in der Reaktion eingesetzte HMPT sind krebserregend, so dass dieses Verfahren aufgrund der möglichen Verunreinigungen für die Herstellung von Nahrungsergänzungsmitteln ungeeignet ist.

WO2012/003625 beschreibt die Herstellung von Hydroxytyrosol durch Ozonolyse von Eugenol bei tiefer Temperatur und anschließende Reduktion des erhaltenen Produkts. Anschließend erfolgt die Demethylierung mit Hilfe einer Lewissäure und eines Mercaptans. Die Tieftemperaturozonolyse ist ein teurer Reaktionsschritt, bei dem Nebenreaktionen wie Oxidation der phenolischen Gruppe nicht auszuschließen sind. Die Demethylierung mit Hilfe extrem übelriechender Stoffe wie Mercaptanen, die zudem nicht einfach ist, macht die Herstellung von Produkten für die Verwendung als Nahrungsergänzungsmittel schwierig. Beide Reaktionsschritte liefern offensichtlich verunreinigte Produkte, die als rotes Öl beschrieben werden.

WO 2012/006783 A1 beschreibt die Herstellung von Hydroxytyrosol ausgehend von preisgünstigem Brenzcatechin, das nach Schutz der phenolischen Gruppen halogeniert wird. Das halogenierte geschützte Brenzcatechin wird anschließend mit Magnesium zur entsprechenden Grignardverbindung umgesetzt, die mit Ethylenoxid zur Reaktion gebracht wird, um die Hydroxyethylgruppe in den aromatischen Ring einzuführen. Die Demethylierung erfolgt wiederum mit Hilfe von Ethanthiol (Ethylmercaptan). Aluminiumchlorid oder Hydrogenolyse von Benzylethern mit Hilfe von Pd/C und H₂.

Bei allen drei beschriebenen Verfahren besteht ein erheblicher Reinigungsaufwand für jede der drei Stufen. Die Ethoxylierung erfolgt zudem mit erheblichem Überschuss an Ethylenoxid, eine Bildung von oligomeren Glycoleinheiten ist daher wahrscheinlich. Die Demethylierung erfolgt je nach Schutzgruppe durch Lewissäure und Ethylmercaptan mit den bereits beschriebenen Problemen oder durch Hydrogenolyse. Die Ausbeuten an Hydroxytyrosol liegen in den drei beschriebenen Verfahren bei 32% bis 70%. Die erhaltenen Produkte sind gelbe bis rote Öle, was auf deutliche Verunreinigungen schließen lässt. Mögliche Verunreinigungen durch Spuren von krebserregendem Ethylenoxid sind für die Verwendung im Nahrungsergänzungsbereich problematisch,

Der Abstract zu CN102344344 beschreibtein ein Verfahren bei dem 3,4-Dialkoxyphenylessigsäurealkylester (Alkyl C1 - C5 und Benzyl) mit Hilfe von Natrium in Alkoholen in einem Schritt reduziert und demethyliert werden. Der Vorteil des Verfahrens ist die Eintopfreaktion, jedoch ist bekannt, dass die Spaltung von Arylethern mit Hilfe von Natrium in Alkoholen mit erheblicher Nebenproduktbildung verbunden ist, da auch eine Spaltung des Arylethers zwischen Sauerstoff und aromatischem Ring auftritt. In den Beispielen liegt die Ausbeute bei max. 50%. Bei allen Beispielen ist eine säulenchromatographische Reinigung erforderlich.

Der Abstract zu CN101891595 beschreibt ein sehr aufwendiges Vierstufenverfahren zur Herstellung von Hydroxytyrosol mit nicht bekanntem Reinigungsaufwand und Verunreinigungen.

Die reduktive Spaltung von 2,2-Dialkyl-1,3-benzdioxolderivaten mit Hilfe von Diisobutylaluminiumhydrid wird von G. Schill et al.; Chem. Ber. 113, 3697 - 3705 1980 beschrieben. Zur Spaltung der Catecholacetale wird im Molverhältnis die mehrals 13-fache Menge Diisobutylaluminiumhydrid verwendet.

In A. Gambacorta, D. Tofani, A. Migliorini; Molecules 2007, 12, 1762-1770 wird eine dreistufige Hydroxytyrosolsynthese beschrieben, die von 3,4-Dihydroxyphenyl-essigsäuremethylester ausgeht. Das Verfahren ist aufwendig und 3,4-Dihydroxy-phenylessigsauremethylester ist keine handelsübliche Substanz, die gemäß dieser Literaturstelle selbst in einem Mehrstufenverfahren hergestellt werden muss.

Aufgabe der vorliegenden Erfindung war es, ein effektives und kostengünstiges Verfahren zur Verfügung zu stellen, welche es ermöglicht, Hydroxytyrosol einfach und in hoher Reinheit herzustellen.

Diese Aufgabe wird gelöst durch ein Verfahren bei dem eine Verbindung der allgemeinen Formel (1) 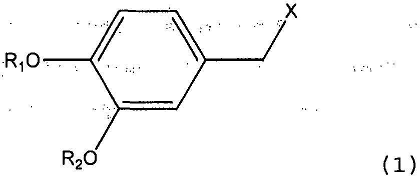 wobei X CH₂OH oder CH₂OM (M = Li, Na, K, Mg, Ca) bedeutet, R₁ und R₂ gleich oder verschieden sind und Alkylrest mit 1 bis 8 Kohlenstoffatomen, Benzylrest, alkyl- oder halogensubstituierter Benzylrest oder Arylalkylrest bedeuten,
wobei R₁ und R₂ auch über 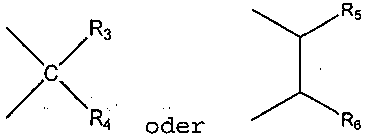 zu einem Ring verknüpft sein können,
R₃, R₄, R₅ und R₆ gleich oder verschieden sind und Wasserstoff- oder Alkylrest mit 1 bis 6 Kohlenstoffatomen, Arylrest, alkyl-substituierter Arylrest bedeuten wobei R₅ und R₆ auch über -(CH₂)₄-, -(CH₂)₅-, oder (CH₂)₆- zu einem Ring verknüpft sein können,
mit einer Aluminiumverbindung der Formel (2)

AlR₇R₈R₉ (2)

umgesetzt wird, wobei R₇, R₈ und R₉ gleich oder verschieden sind und H- oder Alkylrest mit 1 bis 8 Kohlenstoffatomen bedeuten, und anschließend
eine wässrige Lösung einer Hydroxycarbonsäure in einer Menge zugegeben wird, dass eine klare homogene saure Lösung mit einem pH <3 entsteht,
Hydroxytyrosol mit Hilfe eines organischen Lösemittels aus dieser wässrigen klaren homogenen sauren Lösung extrahiert wird und das organische Lösemittel entfernt wird.

Im erfindungsgemäßen Verfahren herrschen während der gesamten Reaktion reduzierende Bedingungen. Somit kann eine Oxidation des empfindlichen Hydroxytyrosols nicht auftreten. Weiterhin treten unter diesen Bedingungen keine Nebenreaktionen, wie Abspaltung von Wasser aus der 2-Phenylethanol-Gruppe auf. Das aus diesem Prozess erhaltene Hydroxytyrosol ist eine farblose klare Flüssigkeit.

Bevorzugt bedeutet X CH₂OH.

Bevorzugt sind R₁, R₂ gleich oder verschieden und bedeuten Alkylrest mit 1 oder 2 Kohlenstoffatomen.

Der alkylsubstituierter Benzylrest ist vorzugsweise ein mit einem Methylrest in Position 2, 3, oder 4 substituierter Benzylrest.

Bei dem Arylalkylrest handelt es sich beispielsweise um einen Phenylethyl-, Phenylpropyl-, Tolylethylrest.

Bevorzugt sind R₃, R₄ R₅ und R₆ gleich oder verschieden und bedeuten Wasserstoff- oder Alkylrest mit 1 oder 2 Kohlenstoffatomen.

Der alkyl-substituierter Arylrest ist vorzugsweise mit einem Methylrest in Position 2, 3, oder 4 substituiert.

Bei dem Arylalkylrest handelt es sich beispielsweise um einen Phenylethyl-, Phenylpropyl-, Tolylethylrest.

Bei der Verbindung der Formel (1) handelt es sich vorzugsweise um eine Verbindung ausgewählt aus der Gruppe 2-(3,4-Dialkoxy)phenylethanol, 2-(3,4-Methylendioxyphehyl)ethanol, 2-(2,2-Dialkylbenzo-[1,3]-dioxol-5-yl)-ethanol, 2,3-Dihydro-1,4-benzodioxin-6-yl)ethanol sowie den Salzen der vorgenannten Alkohole.

Besonders bevorzugt handelt es sich um 2-(3,4-Dimethoxy)phenylethanol, oder 2-(3,4-Methylendioxyphenyl)ethanol.

Bei der Verbindung der Formel (2) handelt es sich vorzugsweise um Diisobutylaluminumhydrid oder Triisobutylaluminium.

Die Reaktion erfolgt bevorzugt bei einer Temperatur zwischen 0° und 200°C bezogen auf einen Druck von 1013 hPa, besonders bevorzugt zwischen 20° und 170°C bezogen auf einen Druck von 1013 hPa.

Die Reaktion erfolgt bevorzugt über einen Zeitraum von 1 bis 25 Stunden, besonders bevorzugt über einen Zeitraum von 5 bis 20 Stunden.

Vorzugsweise werden die Verbindungen der Formel (1) und die Verbindungen der Formel (2) in einem molaren Verhältnis Verbindung Formel (1):Verbindung Formel (2) von 1:3 bis 1:6 eingesetzt. Bevorzugt 1:3 bis 1:4. Wird die Verbindung der Formel (1) im Unterschuss eingesetzt, also weniger als 1:3 mol der Verbindung 1, bezogen auf die 1 mol Verbindung 2, so treten als Reaktionsprodukte lediglich die beiden Monoether des Hydroxytyrosols, (2-(3-Hydroxy-4-alkoxy)phenylethanol und 2-(4-Hydroxy-3-alkoxy)phenylethanol sowie der nicht umgesetzte Diether (2-(3,4-Dialkoxy)phenyl-ethanol) auf. Bei Umsetzung von (2-(3,4-Dimethoxy)phenyl-ethanol) treten als Beiprodukte nur 2-(3-Hydroxy-4-methoxy)phenylethanol und 2-(4-Hydroxy-3-methoxy)phenylethanol auf. Diese Verbindungen sind auch in den natürlichen Olivenextrakten bzw. natürlichem Olivenöl enthalten oder treten als Metaboliten des Hydroxytyrosols auf.

Die Umsetzung der Verbindung der Formel (1) mit einer Aluminiumverbindung der Formel (2) erfolgt vorzugsweise in einem organischen Lösemittel. Geeignete Lösemittel für die Reaktion sind aliphatische oder aromatische Kohlenwasserstoffe, die linear, verzweigt oder cyclisch sein können. Vorzugsweise handelt es sich um einen aromatischer Kohlenwasserstoffe. Insbesondere bevorzugt sind Toluol, Xylol (alle Isomere), Ethylbenzol, Diethylbenzol (alle Isomere), 1,3,5-Trimethylbenzol, Propylbenzol, Isopropylbenzol (Cumol), Butylbenzol oder cyclische Alkylbenzole wie Indan,, C₁ oder C₂ Alkylnaphthaline oder teilhydrierte Naphthaline wie z.B. Tetralin.

Teil des erfindungsgemäßen Verfahrens ist ein einfaches Aufarbeitungsverfahren der erhaltenen Reaktionsmischung, das ohne aufwendige Reinigungsschritte auskommt und direkt zu reinem Hydroxytyrosol führt. Es zeigte sich überraschenderweise, dass sich bei einer Aufarbeitung der erhaltenen Reaktionsmischung mit Hilfe einer wässrigen Lösung einer Hydroxycarbonsäure das Hydroxytyrosol anschließend mit Hilfe eines organischen Extraktionsmittels aus der wässrigen Säurephase auf übliche Weise praktisch quantitativ extrahieren lässt.

Zunächst wird die Reaktionsmischung vorzugsweise nach beendeter Reaktion mit einer wässrigen Lösung einer Hydroxycarbonsäure versetzt.

Bei der wässrigen Lösung einer Hydrocarbonsäure handelt es sich bevorzugt um eine wässrige Lösung von Citronensäure, Äpfelsäure, Milchsäure, Glycolsäure oder Weinsäure.

Vorzugweise enthält die wässrige Lösung Hydroxycarbonsäure in einer Konzentration von 5 bis 50 % (v/v).

Dabei kommt es zur Überführung des Hydroxytyrosols aus der organischen in die wässrige Phase, wobei hydrophobe Verunreinigungen in der organischen Phase verbleiben.

Anschließend erfolgt die Extraktion des Hydroxytyrosols aus der wässrigen Phase mittels eines organischen Lösemittels. Dadurch werden die Aluminiumsalze vom Hydrpxytyrosol abgetrennt. Bei dem Extraktionsmittel handelt es sich vorzugweise um Ether, Carbonsäureester, Carbonsäureamide, Acetale, Ketale, Alkohole oder Alkylamine. Bevorzugt sind Ether oder Carbonsäureester. Besonders geeignet sind dabei Verbindungen, die ein Azeotrop mit Wasser bilden, das einen Siedepunkt < 100°C hat so dass das in der organischen Phase enthaltene Wasser beim Abdestilieren des Lösemittels entfernt wird.

Insbesondere bevorzugt werden Carbonsäurester wie Ethylacetat, Methylacetat, Isopropylacetat eingesetzt, besonders bevorzugt Ethylacetat.

Nach Entfernen des Extraktionsmittels z.B. durch Destillation, erhält man Hydroxytyrosol in hohen Ausbeuten, worunter vorzugsweise Ausbeuten > 80% besonders bevorzugt Ausbeuten > 90% zu verstehen sind, und in hoher Reinheit.

Verbindungen der Formel (1) sind käuflich erhältlich oder können durch übliche Reduktionsverfahren aus handelsüblichen Rohstoffen wie 2-(3,4-Methylendioxyphenyl)essigsäure, 2-(3,4-Dimethoxyphenyl)essigsäure sowie deren Alkylester (Alkyl C1-C4 auch verzweigt) sowie Benzylester hergestellt werden.

Die Verbindungen der Formel (1) können auch in situ erzeugt und sofort zu Hydroxytyrosol weiter umgesetzt werden. In diesem Fall werden im erfindungsgemäßen Verfahren statt Verbindungen der Formel (1) solche der Formel (3) eingesetzt, 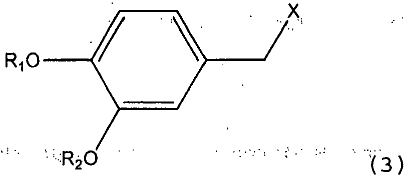 wobei X COOR₁₀ bedeutet,
R₁ und R₂ die für Verbindungen der Formel (1) genannte Bedeutung haben und R₁₀ H-, C₁ bis C₄ Alkylrest, Benzylrest, bevorzugt H-, Methyl-, Ethylrest bedeutet.

Vorzugsweise handelt es sich bei den Verbindungen der Formel (3) um 2-(3,4-Dialkoxy)phenylessigsäure, 2-(3,4-Methylendioxyphenyl)-essigsäure, 2-(2-Alkylbenzo-[1,3]-dioxol-5-yl)-essigsäure, 2,3-Dihydro-1,4-benzodioxin-6-yl)essigsaure, und C₁ bis C₄ Alkyl oder Benzylester der vorgenannten Säuren.

Besonders bevorzugt handelt es sich um 2-(3,4-Dialkbxy)phenylessigsäure oder deren Methyl- oder Ethylester sowie 2-(3,4-Methylendioxyphenyl)-essigsäure oder deren Methyl- oder Ethylester.

Reduktion und Dealkylierung erfolgen hier entweder in einem Schritt mit Verbindungen der Formel (2), wobei mindestens einer der Reste (R₇, R₈ oder R₉) H bedeutet, wie z.B. Diisobutylaluminiumhydrid, oder mit zwei unterschiedlichen Reagenzien sequentiell in einem Eintopfverfahren. In zweitgenannten Fall erfolgt zunächst der Reduktionsschritt mit Reduktionsmitteln wie z.B. Lithiumaluminiumhydrid, Natriumborhydrid, Diisobutylaluminiumhydrid, Alkoxyaluminaten wie Natrium-bis-(2-methoxy-ethoxy)-aluminium-dihydrid zu den Verbindungen der Formel (1) und Dealkylierung erfolgt wie für Verbindungen der Formel (1) beschrieben mit einer Verbindung der Formel (2). Reduktion und Dealkylierung können im gleichen Lösemittel erfolgen, die Reduktion kann aber auch in einem anderen Lösemittel als die Dealkylierung erfolgen, wobei ein Lösemitteltausch ohne Isolierung des intermediär erhaltenen Stoffes der Formel (1) bzw. dessen Salzes möglich ist. Im erstgenannten Fall werden vorzugsweise die für das erfindungsgemäße Verfahren bereits genannten Lösemittel eingesetzt, im zweitgenannten Fall werden Lösemittel wie Ether z.B. Diethylether, Diisopropylether, Dibutylether, 1,2-Dimethoxyethan, auch cyclisch wie z.B. Tetrahydrofuran, Methyltetrahydrofuran, eingesetzt. Die weitere Aufarbeitung erfolgt wie bereits beschrieben.

Das erfindungsgemäße Verfahren liefert mit einer geringen Anzahl von Syntheseschritten eine hohe Ausbeute an isoliertem Hydroxytyrosol. Unter hoher Ausbeute ist dabei vorzugsweise eine Ausbeute von > 80%, bevorzugt > 90% zu verstehen.

Alle im Verfahren vorzugsweise verwendeten Materialien sind käuflich jederzeit preisgünstig verfügbar, insbesondere vorteilhaft ist der Einsatz der kommerziell preiswert verfügbaren Ausgangsstoffe, 2-(3,4-Dimethoxy-phenyl)essigsäure, sowie der Ester der 2-(3,4-Dimethoxyphenyl)-essigsäure und deren Umsetzung in einem Syntheseschritt zu Hydroxytyrosol.

Die folgenden Beispiele dienen der weiteren Beschreibung der Erfindung.

### Beispiele:

### a) die Verbindungen der Formel (1) werden als solche eingesetzt

### Beispiel 1:

In einer Apparatur mit Dreihalskolben, Rührer, Innenthermometer, Dosiertrichter, Rückflußkühler und Inertgasanschluss werden 25g (137 mmol) 2-(3,4-Dimethoxyphenyl)ethanol in 56g Cumol suspendiert und bis auf Rückflusstemperatur erhitzt, wobei 481g einer 21%igen Triisqbutylaluminiumlösung in Cumol innerhalb von 6 Stunden zu dosiert werden. Die Reaktionsmischung wird insgesamt 14 h unter Rückfluss erhitzt.
Nach Abkühlen wird die Reaktionsmischung in 256 g 41,8%-ige wässrige Citronensäurelösung eingetragen. Die organische Phase wird werworfen und die wässrige Phase wird mit 150g Pentan und anschließend mehrfach mit Ethylacetat extrahiert. Die Pentanphase wird verworfen und die Ethylacetatphasen werden vereinigt mit 50g Phosphatpuffer pH 7 gewaschen und das Ethylacetat durch Destillation entfernt.
Ausbeute 19,5g Hydroxytyrosol 92% der Theorie

### Beispiel 2:

5g (27,4 mmol) 3,4-Dimethoxyphenylethanol werden in 13 ml Cumol suspendiert und 79g 21%ige (= 116 mmol) Diisobutylaluminiumhydrid-Lösung in Cumol in 20 min. unter Kühlen (Temp. < 30°C) zu dosiert, wobei eine klare Lösung entsteht. Die Reaktionsmischung wird 5h auf 150°C erhitzt. Eine Probennahme zeigt einen Umsatz von 91,8%. Nach weiteren 3,5h bei 150°C lässt man den Ansatz abkühlen und dosiert den Ansatz unter Eiskühlung zu 59g 40%ige wässriger Citronensäurelösung. Die Phasen werden getrennt, die organische Phase verworfen und die wässrige Phase mit 30 ml Pentan gewaschen. Die Pentanphase wird verworfen und die wässrige Phase anschließend 4 Mal mit 50 ml Ethylacetat extrahiert. Die Ethylacetatphasen werden vereinigt und einmal mit 30g Wasser gewaschen. Danach wird das Ethylacetat durch Destillation entfernt.
Ausbeute: 3,85g 91,1% Hydroxytyrosol

### b) die Verbindungen der Formel (1) werden in situ aus Verbindungen der Formel (3) erzeugt

### Beispiel 3:

In einer Apparatur mit Dreihalskolben, Rührer, Innenthermometer, Dosiertrichter, Rückflusskühler und Inertgasanschluss werden 4,0g (19 mmol) 2-(3,4-Dimethoxyphenyl)-essigsäuremethylester in 4g Xylol gelöst und 13,5g (95 mmol) Diisobutylaluminiumhydrid gelöst in 40,5g Xylol zu dosiert. Nach beendeter Zugabe wird die Reaktionsmischung zum Rückfluss erhitzt. Nach 20 h Rückfluss lässt man die Mischung abkühlen und versetzt sie unter Kühlen mit 105g 20%iger Citronensäurelösung. Die Xylolphase wird verworfen und die wässrige Phase einmal mit Pentan und dreimal mit je 50 ml Ethylacetat extrahiert. Die Pentanphase wird verworfen, die Ethylacetatextrakte werden vereinigt und das Ethylacetat im Vakuum entfernt. Man erhält 2,95g (88% der Theorie)Hydroxytyrosol als klares farbloses Öl.

### Beispiel 4:

Die Durchführung erfolgt analog Beispiel 3 mit dem Unterschied, dass als Lösemittel Diethylbenzol verwendet wird und die Reaktionstemperatur 160°C beträgt. Nach 4h wird der Ansatz wie unter Beispiel 3 beschrieben aufgearbeitet. Ausbeute: 2,8g (93%) Hydroxytyrosol als klares farbloses Öl

### Beispiel 5

2,1g (10 mmol) 2-(3,4-Dimethoxyphenyl)-essigsauremethylester werden in 30 ml Toluol gelöst und 10 ml einer 1M Lösung des Lithiumaluminiumhydrid-Tetrahydrofurankomplexes in Toluol zugegeben. Nach 1,5h Reaktion von 38°C bis 50°C wird die Reaktionsmischung mit 40 ml Diethylbenzol versetzt und Tetrahydrofuran und Toluol abdestilliert, bis die Siedetemperatur 150°C erreicht. Nach Abkühlen werden 18g einer 30%igen Lösung von Diisobutylaluminiumhydrid in Diethylbenzol zugegeben und die Reaktionsmischung anschließend 5,5h auf 150°C erhitzt. Die Aufarbeitung erfolgt wie in Beispiel 3 beschrieben. Ausbeute 82%

### Vergleichsbeispiel 1

Die Durchführung erfolgt analog Beispiel 3 mit dem Unterschied, dass statt mit Citronensäurelösung mit 5%iger Salzsäure aufgearbeitet wurde. Die wässrige Phase bildet ein opakes Gel, aus dem kein Hydroxytyrosol isoliert werden kann.

## Patentansprüche

1. Verfahren zur Herstellung von Hydroxytyrosol **dadurch gekennzeichnet, dass** eine Verbindung der allgemeinen Formel (1) 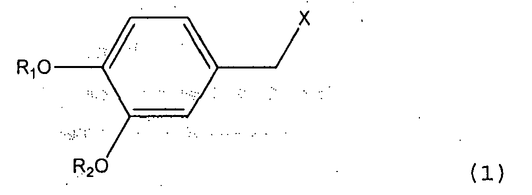 wobei X CH₂OH oder CH₂OM (M = Li, Na, K, Mg, Ca) bedeutet, R₁ und R₂ gleich oder verschieden sind und Alkylrest mit 1 bis 8 Kohlenstoffatomen, Benzylrest, alkyl- oder halogensubstituierter Benzylrest oder Arylalkylrest bedeuten, wobei R₁ und R₂ auch über 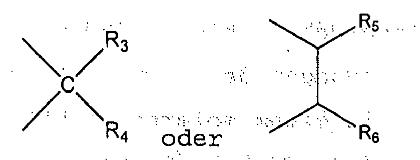 zu einem Ring verknüpft sein können,
R₃, R₄, R₅ und R₆ gleich oder verschieden sind und Wasserstoff- oder Alkylrest mit 1 bis 6 Kohlenstoffatomen, Aryl-rest, alkyl-substituierter Arylrest bedeuten wobei R₅ und R₆ auch über - (CH₂)₄-, -(CH₂)₅-, oder -(CH₂)₆-zu einem Ring verknüpft sein können,
mit einer Aluminiumverbindung der Formel (2)
AlR₇R₈R₉ (2)
umgesetzt wird, wobei R₇, R₈ und R₉ gleich oder verschieden sind und H- oder Alkylrest mit 1 bis 8 Kohlenstoffatomen bedeuten, und anschließend
eine wässrige Lösung einer Hydroxycarbonsäure in einer Menge zugegeben wird, dass eine klare homogene saure Lösung mit einem pH <3 entsteht,
Hydroxytyrosol mit Hilfe eines organischen Lösemittels aus dieser wässrigen klaren homogenen sauren Lösung extrahiert wird und das organische Lösemittel entfernt wird.

2. Verfahren nach Anspruch 1, **dadurch gekennzeichnet, dass** die Verbindung der allgemeinen Formel (1) ausgewählt ist aus der Gruppe 2-(3,4-Dialkoxy)phenylethanol, 2-(3,4-Methylendioxyphenyl)ethanol, 2-(2,2-Dialkylbenzo-[1,3]-dioxol-5-yl)-ethanol, 2,3-Dihydro-1,4-benzodioxin-6-yl)ethanol sowie den Salzen der vorgenannten Alkohole.

3. Verfahren nach Anspruch 1 oder 2, **dadurch gekennzeichnet, dass** die Verbindung der allgemeinen Formel (2) ausgewählt ist aus der Gruppe Diisobutylaluminumhydrid, Triisobutylaluminium.

4. Verfahren nach einem der Ansprüche 1 bis 3, **dadurch gekennzeichnet, dass** die Verbindungen der Formel (1) und die Verbindungen der Formel (2) in einem molaren Verhältnis Verbindung Formel (1):Verbindung Formel (2) von 1:3 bis 1:6 eingesetzt werden, bevorzugt 1:3 bis 1:4

5. Verfahren nach einem der Ansprüche 1 bis 4, **dadurch gekennzeichnet, dass** die Reaktion bei einer Temperatur zwischen 0° und 200°C, bezogen auf einen Druck von 1013 hPa, über einen Zeitraum von 1 bis 25 Stunden, erfolgt.

6. Verfahren nach einem der Ansprüche 1 bis 5, **dadurch gekennzeichnet, dass** die Reaktion der Verbindung der allgemeinen Formel (1) mit der Aluminiumverbindung der allgemeinen Formel (2) in einem organischen Lösemittel erfolgt.

7. Verfahren nach Anspruch 6, **dadurch gekennzeichnet, dass** das Lösemittel ausgewählt ist aus der Gruppe Toluol, Xylol, Ethylbenzol, Diethylbenzol, 1,3,5-Trimethylbenzol, Propylbenzol, Isopropylbenzoll, Butylbenzol, cyclische Alkylbenzole und teilhydrierte Naphthaline.

8. Verfahren nach einem der Ansprüche 1 bis 7, **dadurch gekennzeichnet, dass** Hydroxycarbonsäure ausgewählt ist aus der Gruppe Citronensäure, Weinsäure, Äpfelsäure und Milchsäure.

9. Verfahren nach einem der Ansprüche 1 bis 8 **dadurch gekennzeichnet, dass** das zur Extraktion verwendete organische Lösemittel ausgewählt ist aus der Gruppe Ether, Carbonsäureester, Carbonsäureamide, Acetale, Ketale, Alkohole und Alkylamine.

10. Verfahren nach einem der Ansprüche 1 bis 9 **dadurch gekennzeichnet, dass** die Entfernung des zur Extraktion verwendeten organischen Lösemittels mittels Destillation erfolgt.

## Claims

1. Process for the preparation of hydroxytyrosol, **characterized in that** a compound of the general formula (1) 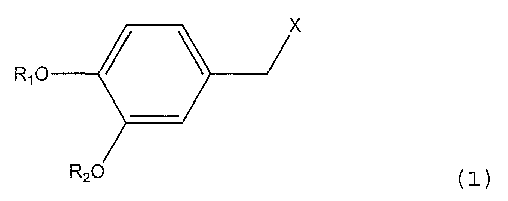 where X is CH₂OH or CH₂OM (M = Li, Na, K, Mg, Ca),
R₁ and R₂ are identical or different and are alkyl radical having 1 to 8 carbon atoms, benzyl radical, alkyl- or halogen-substituted benzyl radical or arylalkyl radical,
where R₁ and R₂ can also be linked via 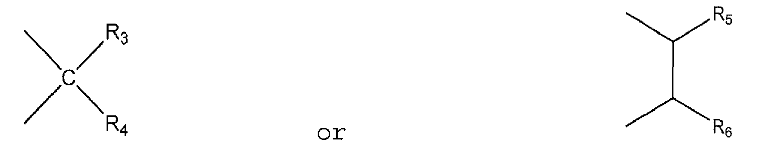 to give a ring,
R₃, R₄, R₅ and R₆ are identical or different and are hydrogen or alkyl radical having 1 to 6 carbon atoms, aryl radical, alkyl-substituted aryl radical,
where R₅ and R₆ can also be linked via - (CH₂)₄-, -(CH₂)₅- or -(CH₂)₆- to give a ring,
is reacted with an aluminum compound of the formula (2)
AlR₇R₈R₉ (2)
where R₇, R₈ and R₉ are identical or different and are H or alkyl radical having 1 to 8 carbon atoms, and then
an aqueous solution of a hydroxycarboxylic acid is added in an amount such that a clear homogeneous acidic solution with a pH < 3 is formed,
hydroxytyrosol is extracted from this aqueous clear homogeneous acidic solution with the help of an organic solvent and the organic solvent is removed.

2. Process according to Claim 1, **characterized in that** the compound of the general formula (1) is selected from the group 2-(3,4-dialkoxy)phenylethanol, 2-(3,4-methylenedioxyphenyl)ethanol, 2-(2,2-dialkylbenzo[1,3]-dioxol-5-yl)ethanol, 2,3-dihydro-1,4-benzodioxin-6-yl)ethanol, and the salts of the aforementioned alcohols.

3. Process according to Claim 1 or 2, **characterized in that** the compound of the general formula (2) is selected from the group diisobutylaluminum hydride, triisobutylaluminum.

4. Process according to any one of Claims 1 to 3, **characterized in that** the compounds of the formula (1) and the compounds of the formula (2) are used in a molar ratio of compound of formula (1): compound of formula (2) of 1:3 to 1:6, preferably 1:3 to 1:4.

5. Process according to any one of Claims 1 to 4, **characterized in that** the reaction takes place at a temperature between 0° and 200°C, based on a pressure of 1013 hPa, over a period of 1 to 25 hours.

6. Process according to any one of Claims 1 to 5, **characterized in that** the reaction of the compound of the general formula (1) with the aluminum compound of the general formula (2) takes place in an organic solvent.

7. Process according to Claim 6, **characterized in that** the solvent is selected from the group toluene, xylene, ethylbenzene, diethylbenzene, 1,3,5-trimethylbenzene, propylbenzene, isopropylbenzene, butylbenzene, cyclic alkylbenzenes and partially hydrogenated naphthalenes.

8. Process according to any one of Claims 1 to 7, **characterized in that** hydroxycarboxylic acid is selected from the group citric acid, tartaric acid, malic acid and lactic acid.

9. Process according to any one of Claims 1 to 8, **characterized in that** the organic solvent used for the extraction is selected from the group ethers, carboxylic acid esters, carboxylic acid amides, acetals, ketals, alcohols and alkylamines.

10. Process according to any one of Claims 1 to 9, **characterized in that** the removal of the organic solvent used for the extraction takes place by means of distillation.

## Revendications

1. Procédé pour la production d'hydroxytyrosol, **caractérisé en ce qu'**on fait réagir un composé de formule générale (1) 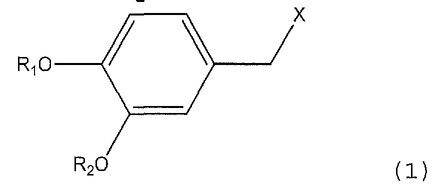 dans laquelle X représente CH₂OH ou CH₂OM (M = Li, Na, K, Mg, Ca),
R₁ et R₂ sont identiques ou différents et représentent un radical alkyle ayant de 1 à 8 atomes de carbone, le radical benzyle, un radical arylalkyle ou benzyle substitué par alkyle ou halogéno,
R₁ et R₂ pouvant également être reliés en un cycle par 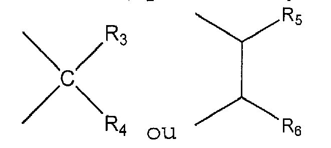 R₃, R₄, R₅ et R₆ étant identiques ou différents et représentant un radical hydrogène ou alkyle ayant de 1 à 6 atomes de carbone, un radical aryle, un radical aryle substitué par alkyle,
R₅ et R₆ pouvant également être reliés en un cycle par - (CH₂)₄, -(CH₂)₅- ou -(CH₂)₆-,
avec un composé d'aluminium de formule (2)
AlR₇R₈R₉ (2),
R₇, R₈ et R₉ étant identiques ou différents et représentant un radical hydrogène ou alkyle ayant de 1 à 8 atomes de carbone, et ensuite
on ajoute une solution aqueuse d'un acide hydroxycarboxylique en une quantité telle qu'il en résulte une solution acide homogène limpide ayant un pH <3,
on extrait l'hydroxytyrosol de cette solution aqueuse acide homogène limpide à l'aide d'un solvant organique et on élimine le solvant organique.

2. Procédé selon la revendication 1, **caractérisé en ce que** le composé de formule générale (1) est choisi dans le groupe constitué par un 2-(3,4-dialcoxy)phényléthanol, le 2-(3,4-méthylènedioxyphényl)éthanol, un 2-(2,2-dialkylbenzo-[1,3]-dioxol-5-yl)-éthanol, le 2,3-dihydro-1,4-benzodioxin-6-yl)-éthanol ainsi que les sels des alcools précités.

3. Procédé selon la revendication 1 ou 2, **caractérisé en ce que** le composé de formule générale (2) est choisi dans le groupe constitué par l'hydrure de diisobutylaluminium, le triisobutylaluminium.

4. Procédé selon l'une quelconque des revendications 1 à 3, **caractérisé en ce qu'**on utilise les composés de formule (1) et les composés de formule (2) en un rapport molaire composé de formule (1):composé de formule (2) de 1:3 à 1:6, de préférence de 1:3 à 1:4.

5. Procédé selon l'une quelconque des revendications 1 à 4, **caractérisé en ce que** la réaction s'effectue à une température comprise entre 0° et 200 °C, sur la base d'une pression de 1 013 hPa, pendant une durée de 1 à 25 heures.

6. Procédé selon l'une quelconque des revendications 1 à 5, **caractérisé en ce que** la réaction du composé de formule générale (1) avec le composé d'aluminium de formule générale (2) s'effectue dans un solvant organique.

7. Procédé selon la revendication 6, **caractérisé en ce que** le solvant est choisi dans le groupe constitué par le toluène, le xylène, l'éthylbenzène, le diéthylbenzène, le 1,3,5-triméthylbenzène, le propylbenzène, l'isopropylbenzène, le butylbenzène, des alkylbenzènes cycliques et des naphtalènes partiellement hydrogénés.

8. Procédé selon l'une quelconque des revendications 1 à 7, **caractérisé en ce que** l'acide hydroxycarboxylique est choisi dans le groupe constitué par l'acide citrique, l'acide d-tartrique, l'acide malique et l'acide lactique.

9. Procédé selon l'une quelconque des revendications 1 à 8, **caractérisé en ce que** le solvant organique utilisé pour l'extraction est choisi dans le groupe constitué par des éthers, des esters d'acides carboxyliques, des carboxamides, des acétals, des cétals, des alcools et des alkylamines.

10. Procédé selon l'une quelconque des revendications 1 à 9, **caractérisé en ce que** l'élimination du solvant organique utilisé dans l'extraction s'effectue par distillation.
